# EUROPEAN PATENT APPLICATION

(11) **EP 4 825 315 A1**
(43) Date of publication of application: **21.10.2026**
(21) Application number: 25852345.5
(22) Date of filing: 11.08.2025
(51) Int. Cl.: A61B 5/256, A61B 5/291, A61B 5/00, A61B 5/265

(54) **BIOSIGNAL SENSOR**

(30) Priority: 09.08.2024 KR 20240107092; 15.04.2025 KR 20250049178
(71) Applicant: Brainu Co., Ltd., Chungju-si, Chungcheongbuk-do 27465 (KR)
(72) Inventor: HONG, Seung Kyun, Gwangmyeong-si, Gyeonggi-do 14233 (KR); KIM, Kwang Moo, Seongnam-si, Gyeonggi-do 13568 (KR); HAN, Chi Hoon, Gwangmyeong-si, Gyeonggi-do 14245 (KR); KIM, Kwan Hong, Seoul 08207 (KR); YOON, Hyung Woo, Seongnam-si, Gyeonggi-do 13397 (KR); LEE, Kyu Hong, Yongin-si, Gyeonggi-do 17046 (KR)
(74) Representative: ABG Intellectual Property Law, S.L.
(86) International application number: PCT/KR2025/012115
(87) International publication number: WO 2026/035121

(57) **Abstract**

The present invention relates to a biosignal sensor, wherein the biosignal sensor collects biosignals, including brain waves, from a hairy body part of an animal, including a human, and includes a fixing band surrounding and fixing a body part, an electrode assembly provided with a plurality of electrodes and disposed on a rear surface of the fixing band, the rear surface being in a direction toward the skin of the animal, and a conductive material storage body that electrically connects the plurality of electrodes to the skin of the animal by means of a conductive material. Accordingly, by electrically connecting the electrode assembly disposed on the forehead of the animal to the skin of the animal via the conductive material storage body, it is possible to improve conductive close-contact between a biosignal-collecting electrode and the skin of the animal.

## Description

### TECHNICAL FIELD

The present invention relates to a biosignal sensor, and more particularly, to a biosignal sensor that collects biosignals, including brain waves, from a hairy body part of an animal, including a human.

### BACKGROUND ART

In general, biosignals are caused by the electrical activity of nerve cells constituting an animal, including a human, and may be measured in the form of electrical signals via an electrode on the skin, and in particular, brain waves are caused by the electrical activity of nerve cells constituting the brain.

Since body parts, head shapes, sizes, curvatures, and the like to be measured vary depending on the target of measurement of biosignals, including brainwaves, the size of a biosignal sensor attached to the skin is required to be miniaturized to maintain stable skin contact of an electrode, and a close-contact state and an electrical connection state are important. In addition, maintaining an appropriate distance between measurement channels is also an important factor for biosignal quality.

In a special situation that requires a surgery and the like, it is necessary to measure brain waves to determine the depth of anesthesia, in which case, the depth of anesthesia may be determined by measuring brain waves of a surgical subject.

In particular, in the case of an animal, there is hair near the frontal lobe to be measured for brainwaves, so that there has been a problem in that the close-contact state between the skin and an electrode is non-uniform.

In addition, since an animal has a soft scalp, even if a biosignal sensor is fixed using a fixing band, the biosignal sensor moves back and forth near the frontal lobe, resulting in a problem in that the position at which the biosignal sensor is fixed changes.

Therefore, there is a need for a biosignal sensor that may be simply and stably fixed to a hairy body part and the head of an animal while being able to stably maintain close-contact with the skin.

### DISCLOSURE OF THE INVENTION

### TECHNICAL PROBLEM

In order to solve the above-described problems of the background technology, the purpose of the present invention is to provide a biosignal sensor that electrically connects an electrode assembly disposed on the forehead of an animal to the skin of the animal via a conductive material storage body, and thus may improve conductive close-contact between a biosignal-collecting electrode and the skin of the animal.

In addition, the purpose of the present invention is to provide the biosignal sensor in which a fixing band is made of a flexible material such as a urethane film, and thus may be stably fixed by flexibly surrounding the head according to a head shape of the animal.

In addition, the purpose of the present invention is to provide the biosignal sensor in which the electrode assembly is provided with an insulating part that insulates a wiring part, and thus may reduce the generation of noise due to contact between a wire and the skin while the biosignal sensor is fixed to and worn on the animal.

In addition, the purpose of the present invention is to provide the biosignal sensor in which the electrode assembly is provided with a connecting part, and thus may facilitate the connection to an amplifier and an external device.

In addition, the purpose of the present invention is to provide the biosignal sensor in which the electrode assembly is coupled to the fixing band by using a double-sided tape or an adhesive, and thus may be simply coupled.

In addition, the purpose of the present invention is to provide the biosignal sensor in which an electrode part and the wiring part are formed by printing silver or silver chloride ink, and thus may facilitate the formation of electrodes and wires, and may facilitate the transmission of electrical signals while maintaining the flexibility of the electrode assembly.

In addition, the purpose of the present invention is to provide the biosignal sensor in which the electrodes are disposed by including a ground electrode, a reference electrode, and a channel electrode, and thus may measure biosignals of the animal while miniaturizing the biosignal sensor.

In addition, the purpose of the present invention is to provide the biosignal sensor in which the ground electrode is disposed at the center and the reference electrode and the channel electrode are disposed on respective sides of the ground electrode at equal distances, and thus may improve the quality of the collected biosignals.

In addition, the purpose of the present invention is to provide the biosignal sensor in which a conductive material is embedded inside the storage part of the conductive material storage body, and thus may improve the electrical connection and close-contact between the electrode and the skin of the animal.

In addition, the purpose of the present invention is to provide the biosignal sensor in which a porous foam that absorbs and stores the conductive material is provided inside the storage part, and thus may minimize the volatilization and evaporation of the conductive material.

In addition, the purpose of the present invention is to provide the biological signal sensor in which a cross-sectional diameter of an opening of the storage part is formed smaller than a cross-sectional diameter of an internal space of the storage part, and thus may maintain a stored state such that the conductive material does not flow out, and may allow the conductive material to be discharged by pumping the storage part when necessary.

In addition, the purpose of the present invention is to provide the biological signal sensor in which a plurality of partition wall portions are formed around each of a plurality of storage parts, and thus may prevent the conductive material stored in each of the storage parts from being mixed, thereby preventing short circuits between the plurality of electrodes.

In addition, the purpose of the present invention is to provide the biological signal sensor in which one or more guide parts are formed at a lower portion of any one of the plurality of storage parts by protruding from the bottom surface of a main body part, and thus may prevent the main body part from slipping from the skin of the animal.

In addition, the purpose of the present invention is to provide the biological signal sensor in which a close-contact pad is provided on an upper surface around the plurality of storage parts, and thus may fix the electrode part at an accurate position on the skin of the animal, the position being suitable for collecting the biosignals.

### TECHNICAL SOLUTION

A biosignal sensor of the present invention for solving the above-described problems is a biosignal sensor that collects biosignals, including brain waves, from a hairy body part of an animal, including a human, and includes an electrode assembly fixed to the body part and provided with a plurality of electrodes disposed toward the skin of the animal, and a conductive material storage body that electrically connects the plurality of electrodes to the skin of the animal by means of a conductive material.

Preferably, a fixing band surrounding the body part to bring the plurality of electrodes into close contact with the skin of the animal is further included, wherein the electrode assembly is disposed on a rear surface of the fixing band, the rear surface being in a direction toward the skin of the animal.

Preferably, the electrode assembly includes a sheet part coupled to the rear surface of the fixing band, an electrode part disposed on a rear surface of the sheet part and composed of a plurality of electrodes that collect the biosignals of the animal, a wiring part composed of a plurality of wires, each having one end electrically connected to each of the plurality of electrodes, an insulating part that insulates the wiring part, and a connecting part electrically connected to the other end of each of the plurality of wires to electrically transmit the collected biological signals to the outside.

Preferably, the sheet part is detachably attached to the fixing band.

Preferably, the conductive material storage body includes a main body part provided with a coupling surface coupled to a rear surface of the electrode assembly, and a contact surface in contact with the skin of the animal in a direction opposite to the coupling surface, and a plurality of storage parts each disposed at a position corresponding to the position of each of the plurality of electrodes, and provided with a plurality of internal spaces and openings formed through the main body part such that the electrode part is toward the skin of the animal, wherein the conductive material is embedded in each of the internal spaces of the plurality of storage parts.

Preferably, the storage part is made of an elastic material, such that, when elastic deformation occurs due to an external force, the conductive material is discharged from the opening of the storage part onto the skin.

Preferably, the storage portion is formed such that a cross-sectional diameter of the opening is smaller than a cross-sectional diameter of the internal space.

Preferably, a porous foam inserted into the internal space of the storage part, and having a porous structure that absorbs the conductive material to maintain a stored state is further included.

Preferably, one or more partition wall portions formed around the plurality of storage parts by protruding from a bottom surface of the main body part are further included.

Preferably, one or more guide parts formed at a lower portion of any one of the plurality of storage parts by protruding from the bottom surface of the main body part are further included.

Preferably, a plurality of close-contact pads, each having a front surface attached to an upper surface around the opening of each of the storage parts and a rear surface attached to the skin of the animal, are further included.

Preferably, the close-contact pad covers a portion of the opening of the storage part.

As a biosignal sensor of another embodiment of the present invention for solving the above-mentioned problems, the electrode assembly includes an electrode part disposed on the rear surface of the fixing band and composed of a plurality of electrodes that collect the biosignals of the animal, a wiring part disposed on the rear surface of the fixing band, and each having one end electrically connected to each of the plurality of electrodes, an insulating part that insulates the wiring part, and a connecting part electrically connected to the other end of each of the plurality of wires to electrically transmit the collected biological signals to the outside.

### ADVANTAGEOUS EFFECTS

A biosignal sensor of the present invention electrically connects an electrode assembly disposed on the forehead of an animal to the skin of the animal via a conductive material storage body, and thus may improve conductive close-contact between a biosignal-collecting electrode and the skin of the animal.

In addition, in the present invention, a fixing band is made of a flexible material such as a urethane film, and thus, may be stably fixed by flexibly surrounding the head according to a head shape of the animal.

In addition, in the present invention, the electrode assembly is provided with an insulating part that insulates a wiring part, and thus, may reduce the generation of noise due to contact between a wire and the skin while the biosignal sensor is fixed to and worn on the animal.

In addition, in the present invention, the electrode assembly is provided with a connecting part, and thus may facilitate the connection to an amplifier and an external device.

In addition, in the present invention, the electrode assembly is coupled to the fixing band by using a double-sided tape or an adhesive, and thus may be simply coupled.

In addition, in the present invention, an electrode part and the wiring part are formed by printing silver or silver chloride ink, and thus may facilitate the formation of electrodes and wires, and may facilitate the transmission of electrical signals while maintaining the flexibility of the electrode assembly.

In addition, in the present invention, the electrodes are disposed by including a ground electrode, a reference electrode, and a channel electrode, and thus may measure biosignals of the animal while miniaturizing the biosignal sensor.

In addition, in the present invention, the ground electrode is disposed at the center and the reference electrode and the channel electrode are disposed on respective sides of the ground electrode at equal distances, and thus may improve the quality of the collected biosignals.

In addition, in the present invention, a conductive material is embedded inside the storage part of the conductive material storage body, and thus may improve the electrical connection and close-contact between the electrode and the skin of the animal.

In addition, in the present invention, a porous foam that absorbs and stores the conductive material is provided inside the storage part, and thus may minimize the volatilization and evaporation of the conductive material.

In addition, in the present invention, a cross-sectional diameter of an opening is formed smaller than a cross-sectional diameter of an internal space, and thus may maintain a stored state such that the conductive material does not flow out, and may allow the conductive material to be discharged by pumping the storage part when necessary.

In addition, in the present invention, a plurality of partition wall portions are formed around each of a plurality of storage parts, and thus may prevent the conductive material discharged around each of the storage parts from being mixed, thereby preventing short circuits between the plurality of electrodes.

In addition, in the present invention, one or more guide parts are provided, which are formed at a lower portion of any one of the plurality of storage parts by protruding from the bottom surface of a main body part, and thus may prevent the main body part from slipping from the skin of the animal.

In addition, in the present invention, a close-contact pad is provided on an upper surface around the plurality of storage parts, and thus may fix the electrode part at an accurate position on the skin of the animal, the position being suitable for collecting the biosignals.

### BRIEF DESCRIPTION OF THE DRAWINGS

FIG. 1 is a front view of a biosignal sensor according to a first embodiment of the present invention.
FIG. 2 is a rear view of the biosignal sensor according to the first embodiment of the present invention.
FIG. 3 is a front view of a fixing band according to the first embodiment of the present invention.
FIG. 4 is an exploded perspective view of the biosignal sensor according to the first embodiment of the present invention, as viewed from the rear.
FIG. 5 is a rear perspective view of a conductive material storage body constituting the present invention.
FIG. 6 is a front perspective view of the conductive material storage body constituting the present invention.
FIG. 7 is a cross-sectional view taken along line A-A of FIG. 5 constituting the present invention.
FIG. 8 is a cross-sectional view of the biosignal sensor according to the first embodiment of the present invention.
FIG. 9 is a view showing a state in which the biosignal sensor according to the first embodiment of the present invention is worn on the head of an animal.
FIG. 10 is a view showing a state in which the biosignal sensor according to the first embodiment of the present invention is worn on the torso of an animal.
FIG. 11 is a front view of a fixing band according to a second embodiment of the present invention.
FIG. 12 is a rear view of the fixing band according to the second embodiment of the present invention.

### MODE FOR CARRYING OUT THE INVENTION

Hereinafter, embodiments of the present invention will be described in detail with reference to the accompanying drawings. The present invention relates to a biosignal sensor that collects biosignals, including brain waves, from a hairy body part of an animal, including a human.

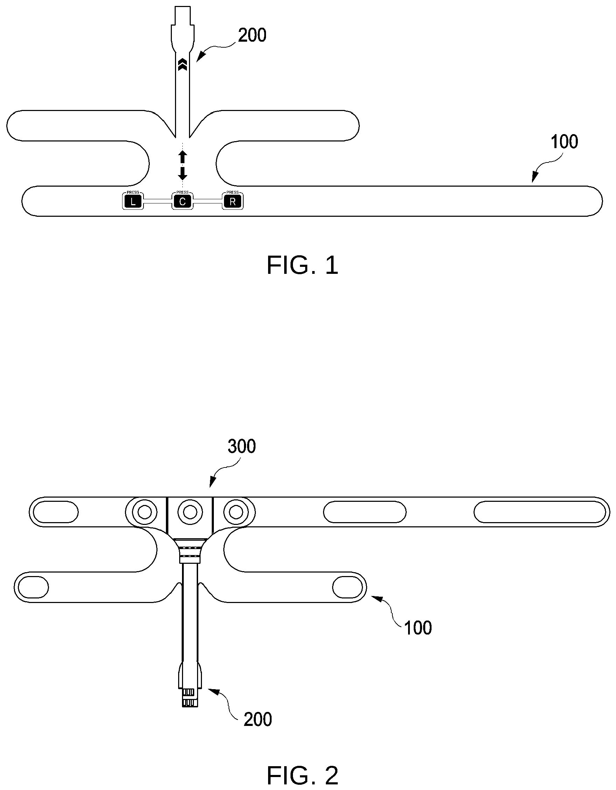

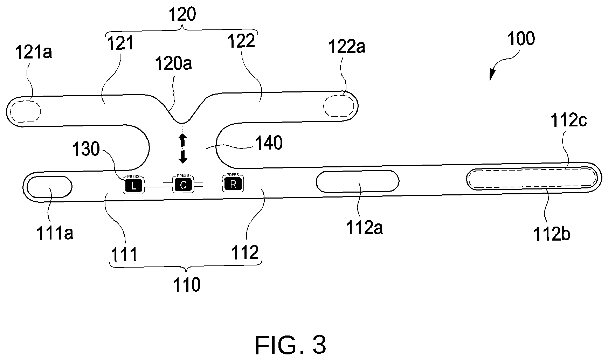

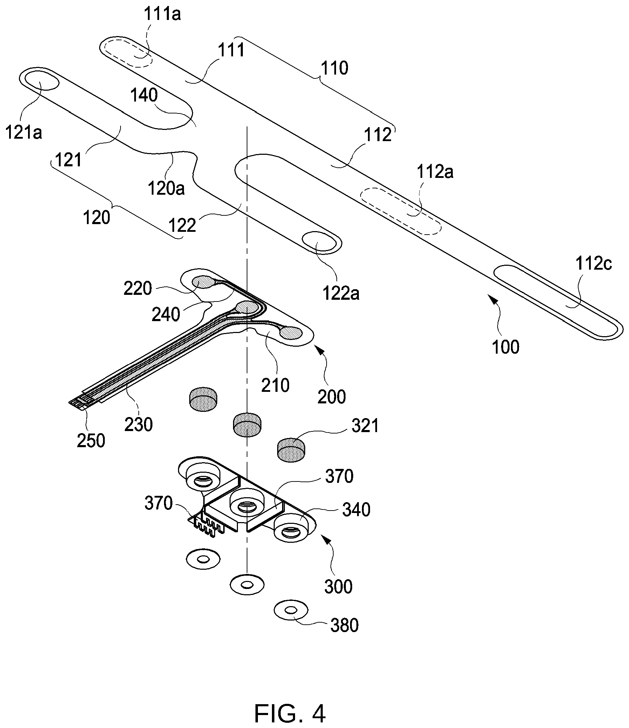

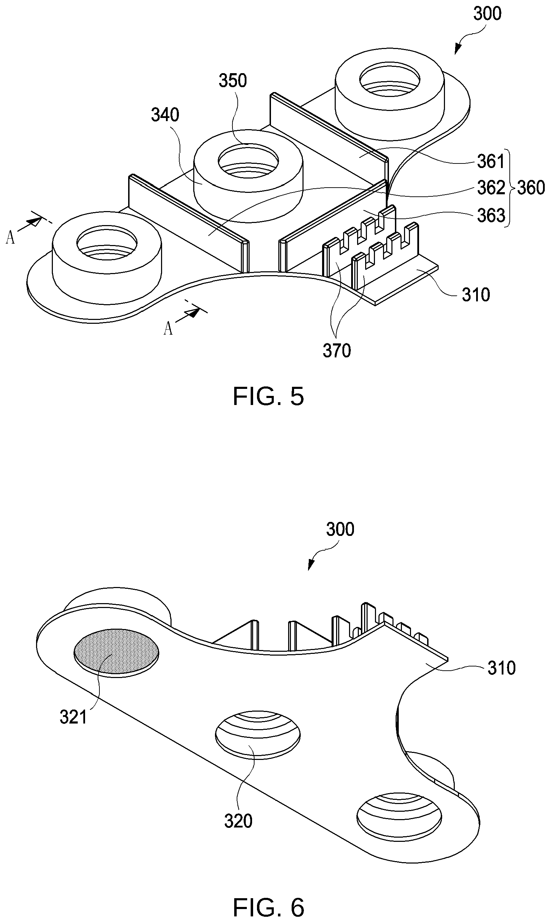

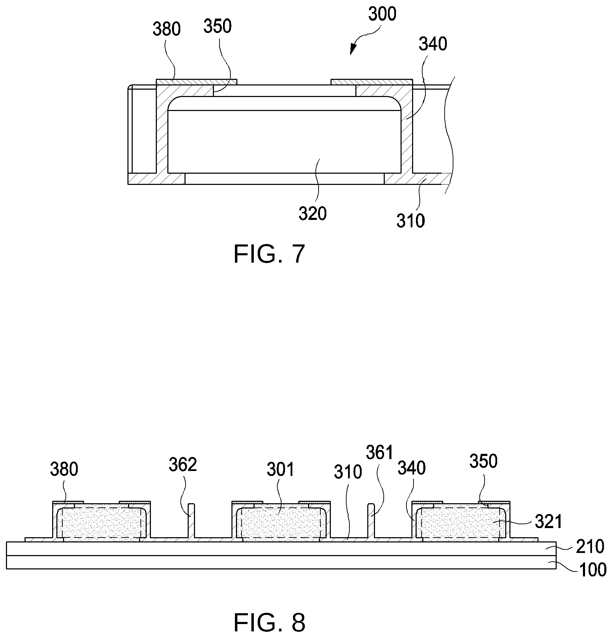

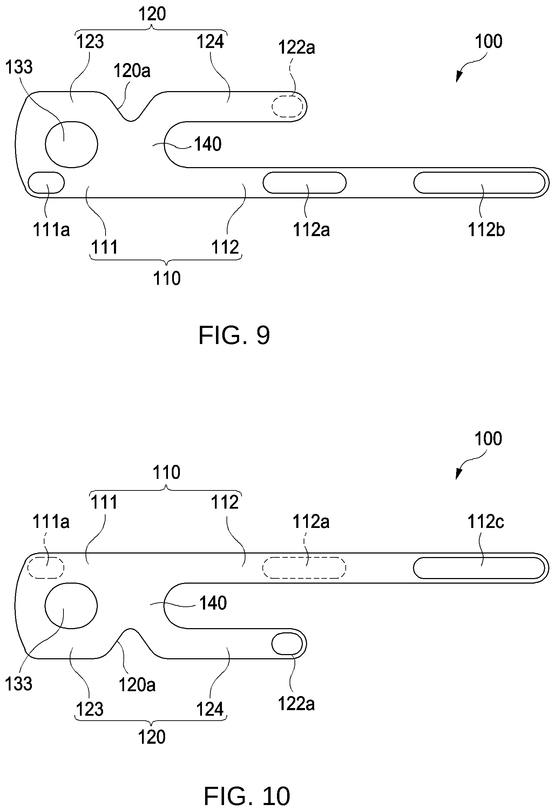

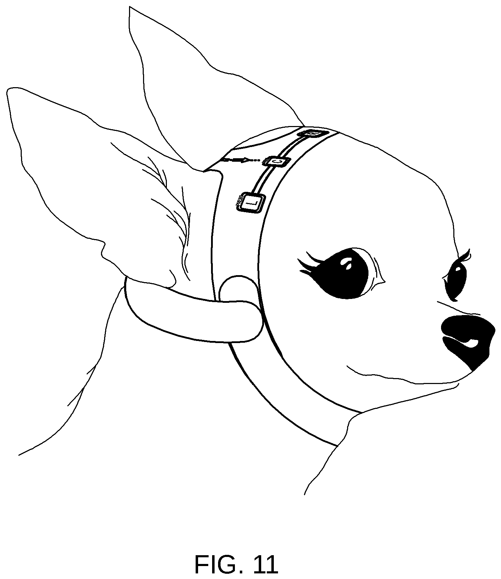

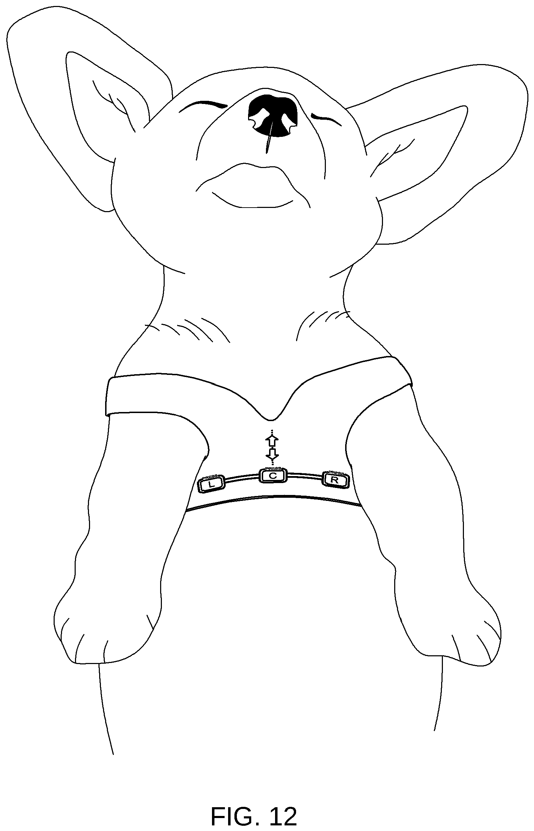

FIG. 1 is a front view of a biosignal sensor according to a first embodiment of the present invention, FIG. 2 is a rear view of a biosignal sensor according to the first embodiment of the present invention, FIG. 3 is a front view of a fixing band according to the first embodiment of the present invention, FIG. 4 is an exploded perspective view of the biosignal sensor according to the first embodiment of the present invention, as viewed from the rear, FIG. 5 is a rear perspective view of a conductive material storage body constituting the present invention, FIG. 6 is a front perspective view of the conductive material storage body constituting the present invention, FIG. 7 is a cross-sectional view taken along line A-A of FIG. 5 constituting the present invention, FIG. 8 is a cross-sectional view of the biosignal sensor according to the first embodiment of the present invention, FIG. 9 is a view showing a state in which an animal wears a biosignal sensor according to the first embodiment of the present invention, FIG. 10 is a view showing a state in which the biosignal sensor according to the first embodiment of the present invention is worn on the torso of an animal, FIG. 11 is a front view of a fixing band according to a second embodiment of the present invention, and FIG. 12 is a rear view of the fixing band according to the second embodiment of the present invention.

The biosignal sensor according to the first embodiment of the present invention, as illustrated in FIG. 1 to FIG. 8, generally includes a fixing band 100, an electrode assembly 200, and a conductive material storage body 300.

The fixing band 100 is a flexible fixing member that is fixed to a hairy body part of an animal, including a human, and is fixed to the body part to collect and measure biosignals, including brain waves, from the skin of the animal. The fixing band 100 is made of a flexible material such as a urethane film, and thus, may be fixed to the body part of the animal by being brought into close contact therewith without causing irritation to the skin of the animal. In particular, the fixing band 100 may be fixed to the head of the animal and used for measuring the brain waves among the biosignals. The fixing band 100 is composed of a head fixing part 110, a connecting part 140, an ear fixing part 120, and a character pattern 130.

The head fixing member 110 is a fixing member that surrounds and fixes the chin and head of the animal, and is formed in the shape of a long belt and fixed by being disposed to wrap around the face of the animal. The head fixing member 110 is composed of a first head fixing member 111 and a second head fixing member 112.

The first head fixing member 111 extends toward one side with respect to the connecting member 140, and may be disposed from the forehead of the animal to a side surface of the face. A first detachable member 111a may be provided at an end of a front surface of the first head fixing member 111. Here, the front surface refers to a direction opposite to a direction toward the skin of the animal.

The second head fixing member 112 extends toward the other side with respect to the connecting member 140, and may be disposed from the forehead of the animal, past along a side surface of the face on the other side and under the chin, to be in contact with the end of the first head fixing member 111. A second detachable member 112a may be provided in the middle of a front surface of the second head fixing member 112, a third detachable member 112b may be provided at an end of the front surface thereof, and a fourth detachable member 112c may be provided at an end of a rear surface thereof. Here, the rear surface refers to the direction toward the skin of the animal.

Thus, the first head fixing part 111 is formed shorter than the second head fixing part 112, and surrounds the head on both sides with respect to the connecting part 140 while compressing and surrounding the head of the animal, wherein each of the ends are coupled to each other and fixed on the side surface of the face.

At this time, the first detachable member 111a on the front surface of the first head fixing member 111 and the fourth detachable member 112c on the rear surface of the second head fixing member 112 may be coupled to each other and fixed. It is preferable that the first and fourth detachable members 111a and 112c are formed of Velcro.

Although not illustrated in the drawings, the shapes and lengths of the first and second head fixing members 111 and 112 may be formed in various ways, and any form is possible as long as they can surround and fix a head, and the first and fourth detachable members 111a and 112c may be of any form as long as they can be coupled to each other.

The connecting part 140 is a connecting member that protrudes and extends in the middle of a longitudinal direction of the head fixing part 110, and connects the head fixing part 110 to the ear fixing part 120. It is preferable that a position from which the connecting part 140 protrudes is at the center of the forehead of the animal.

The ear fixing part 120 is a fixing member that surrounds and fixes each ear of the animal, and is respectively formed on one side and the other side of the connecting part 140 and disposed to surround and wrap around each of an ear on one side and an ear on the other side of the animal. The ear fixing part 120 is composed of a first ear fixing part 121 and a second ear fixing part 122.

The first ear fixing part 121 branches off from an end of the connecting part 140, and extends toward one side to surround and fix the ear on one side of the animal. A fifth detachable member 121a may be provided at an end of a rear surface of the first ear fixing part 121. The first ear fixing part 121 surrounds and wraps around the ear on one side of the animal, and the fifth detachable member 121a may be coupled to the third detachable member 112b. Specifically, while the fourth detachable member 112c on the rear surface of the second head fixing part 112 is coupled to the first detachable member 111a on the front surface of the first head fixing part 111, the fifth detachable member 121a on the rear surface of the first ear fixing part 121 is coupled to the third detachable member 112b on the front surface of the second head fixing part 112.

The second ear fixing part 122 branches off from the end of the connecting part 140, and extends toward the other side to surround and fix the ear on the other side of the animal. A sixth detachable member 122a may be provided at an end of a rear surface of the second ear fixing part 122. The second ear fixing part 122 surrounds and wraps around the ear on the other side of the animal, and the sixth detachable member 122a may be coupled to the second detachable member 112a. Specifically, the sixth detachable member 122a on the rear surface of the second ear fixing part 122 is coupled to the second detachable member 112a disposed on the front surface in the middle of the second head fixing part 112.

Thus, the first ear fixing part 121 and the second ear fixing part 122 are formed to have the same length, and branch off from the end of the connecting part 140 toward respective sides and surround the ears on the respective sides. Thereafter, the ends of the first and second ear fixing parts 121 and 122 are coupled to a detachable member of the head fixing part 110 and fixed. It is preferable that the third and fifth detachable members 112b and 121a and the second and sixth detachable members 112a and 122a are formed of Velcro.

The ear fixing part 120 may have a groove 120a formed concavely toward the connecting part 140 from which the first and second ear fixing parts 121 and 122 branch off. The groove 120a may allow the first and second ear fixing parts 121 and 122 to bend more flexibly to improve close-contact with the skin when they are bent while respectively wrapping around the ears on the respective sides.

Although not illustrated in the drawings, the shape and length of the ear fixing part 120 may be formed in various ways, and any form is possible as long as it can surround and fix an ear. In addition, the third and fifth detachable members 112b and 121a and the second and sixth detachable members 112a and 122a may each be of any form as long as they can be coupled to each other.

Meanwhile, the length of at least one of the detachable members that are coupled to each other may be formed longer than the width thereof. Thus, the length for surrounding and wrapping around the head and around the ears of the animal may be adjusted to a predetermined length.

In the case of an animal, the scalp is soft, so that the electrode assembly 200 may move back and forth on the head of the animal with only the head fixing part 110. Therefore, by forming the ear fixing part 120 and fixing the ears together therewith, the electrode assembly 200 may be stably fixed by the fixing band 100.

The character pattern 130 is a display member printed or displayed on a front surface of the fixing band 100, and guides a user by displaying the position of the electrode assembly, which is disposed at the center of the forehead of the animal and a rear surface.

Thus, the user may position the center of the electrode assembly 200 at the center of the forehead of the animal without having to confirm by turning over the fixing band 100 in order to confirm the position of the electrode assembly 200 disposed on the rear surface of the fixing band 100, and may identify the position of each of the plurality of electrode parts 220 constituting the electrode assembly 200.

The electrode assembly 200 is coupled to the rear surface of the fixing band 100 and disposed on a body part of the animal to collect biosignals including brain waves of the animal, and is composed of a sheet part 210, an electrode part 220, a wiring part 230, an insulating part 240, and a connecting part 250.

The sheet part 210 is a sheet member that is coupled to the rear surface of the fixing band 100, and is preferably made of a flexible material such as urethane. The sheet part 210 may be coupled to the fixing band 100 by using a double-sided tape or an adhesive.

The electrode part 220 is an electrode member that is configured on a rear surface of the sheet part 210 and disposed on the body part of the animal to collect biosignals of the animal, and in the embodiment, is disposed on the head fixing member 110 to be disposed on the forehead of the animal, and is composed of a plurality of electrodes.

The plurality of electrodes are provided in a number of three or more by including a ground electrode, a reference electrode, and a channel electrode, and may improve the quality of biosignal collection. For example, it is preferable that the ground electrode is disposed at the center of the forehead of the animal, and the reference electrode and the channel electrode are disposed on respective sides of the ground electrode spaced apart at equal distances therefrom.

Meanwhile, the closer the electrode is to the frontal lobe, the better the quality of the collected biosignals, but in the case of small animals, it is often difficult to position the electrode on the frontal lobe due to the narrow forehead, so that it is preferable that the electrode part 220 is disposed between the frontal lobe and the crown of the head.

The wiring part 230 is a wiring member that electrically transmits brain wave signals collected from the electrode part 220, and is composed of a plurality of wires, each having one end electrically connected to each of the plurality of electrodes. Each of the plurality of wires is formed so as not to overlap with each other.

It is preferable that the wiring part 230 is disposed in a direction in which it is drawn out from each of the plurality of electrodes toward the crown of the animal.

The electrode part 220 and the wiring part 230 may be formed by printing silver or silver chloride ink on the sheet part 210, and any material and form are possible as long as they can collect biosignals and transmit signals.

Meanwhile, although not illustrated in the drawings, an electrode part and a wiring part may be disposed on the rear surface of a fixing band while a sheet part is not provided. For example, a method of directly printing the electrode part and the wiring part on the rear surface of the fixing band may be used.

The insulating part 240 is an insulating material that wraps around a rear surface of the wiring part 230 to insulate the wiring part 230, and may prevent short circuits from occurring due to contact between the wiring part 230, which passes between the electrode part 220 or is drawn out toward the crown of the head of the animal, with the skin of the animal, the user, an external conductive material, and the like.

The connecting part 250 is a connecting member that electrically connects and transmits the biosignals received from the plurality of wires to the outside, and is electrically connected to the other end of each of the plurality of wires. The connecting part 250 is connected to an external device such as a signal amplifier or collector.

The conductive material storage body 300 is a conductive member that is coupled to a rear surface of the electrode assembly 200 and electrically connects the electrode assembly 200 to the skin of the animal by means of a conductive material 301, and is composed of a main body part 310 and a storage part 320.

The main body part 310 is a main body member that is coupled to the rear surface of the electrode assembly 200, and is provided with a coupling surface coupled to the rear surface of the electrode assembly and a contact surface in contact with the skin of the animal in a direction opposite to the coupling surface. The main body 310 is disposed by including a position corresponding to the plurality of electrodes, and may be formed by partially protruding in a direction toward the crown of the head of the animal in order to improve a contact area and fixing force with the electrode assembly 200. The main body part 310 may be composed of a material such as silicone rubber.

The storage part 320 is disposed at a position corresponding to positions of the plurality of electrodes, and each of the plurality of electrode parts 220 is provided with a plurality of internal spaces and openings formed through the main body part 310 to be in communication with the skin of the animal.

The conductive material 301 is embedded in each of the plurality of internal spaces. The storage part 320 is made of an elastic material, such that, when the user applies pressure to the electrode part 220 by using a finger or the like, elastic deformation occurs, thereby allowing the embedded conductive material 301 to be discharged through the opening, and the discharged conductive material 301 permeates the hair of the animal and reaches the skin. As a result, the conductive material embedded in each internal space electrically connects between the skin of the animal and the electrode part 220.

As the conductive material 301, a gel-state or sol-state material may be used, or a liquid material may be used. Animals other than humans have a large amount of hair even on the forehead portion, thereby making it difficult for the electrode portion 220 to be directly in close contact with the forehead skin of the animal, so that it is preferable that the conductive material 301 has a suitable viscosity to improve conductivity.

At this time, the storage part 320 is formed such that a cross-sectional diameter of the opening is narrower than a cross-sectional diameter of the internal space. Thus, in the absence of an external force, the conductive material 301 stored in the storage part 320 may maintain a stored state without flowing out through the opening due to surface tension.

A porous foam 321 is inserted into the internal space of the storage part 320, and is formed of a porous structure that absorbs and stores the conductive material 301. The porous foam 321 serves as an electrical connection guide that transmits the biosignals of the animal to the electrode part 220. At this time, the porous foam 321 may be formed of a conductive material to further improve conductivity.

A partition wall portion 360 is composed of one or more partition walls 361, 362, and 363 formed around each storage part 320 by protruding from a bottom surface of the main body part 310. Each of the partition walls 361, 362, and 363 is formed at the same height as or slightly higher than the height of the storage part 360 to be in close contact with the skin of the animal in order to prevent an electrical short circuit of the electrode part 220 due to mixing of the conductive material 301 stored in each of mutually adjacent storage parts 320.

Although not displayed in the drawings, the partition wall portion may be formed in a cylindrical shape to completely surround the storage part, or may be formed to have a cross-section in an arc shape in order to surround only a portion of the storage part.

A guide part 370 is composed of one or more brushes formed at a lower portion of any one of the plurality of storage parts 320 by protruding from the bottom surface of the main body part 310. The brush has irregularities formed on an upper surface thereof to perform a function of fixing the main body part 310 to the skin of the animal in order to prevent the main body part 310 from sliding down from the skin of the animal. In particular, the guide part 370 performs a function of improving signal quality by preventing the risk of the electrode part 220 moving due to a reduction in friction force between the main body part 310 and the skin of the animal when a liquid conductive material 301 is discharged.

As shown in FIG. 7, a close-contact pad 380 has a front surface attached to an upper surface around the opening of each of the plurality of storage parts 320, and a rear surface attached to the skin of the animal. The close-contact pad 380 performs a function of fixing the electrode part 220 at an accurate position on the skin of the animal, the position being suitable for collecting biosignals. In addition, when a release film is used for product packaging, the close-contact pad 380 is attached to a surface on the inside of the release film to perform a function of preventing the conductive material 301 from leaking or being exposed to air, thereby being dried.

At this time, the close-contact pad 380 may be formed to cover a portion of the opening of the storage part 320 to further prevent the conductive material 301 from leaking. In addition, the close-contact pad 380 may be formed of a conductive material to further improve biosignal collection capability of the electrode part 220.

Hereinafter, a method for fixing and using the biosignal sensor of the present invention will be briefly described. As illustrated in FIG. 9, a user fixes the head fixing part 110 around the head of the animal, and then fixes the first and second ear fixing parts 121 and 122 around ears on respective sides. At this time, the center (identifiable by the character pattern) of the electrode assembly 200 is adjusted and fixed such that it is positioned at the center of the forehead of the animal.

The connecting part 250 of the electrode assembly 200 is connected to an external device, and when the preparation for biosignal collection is complete, the front surface side of the electrode part 220 is pressed to allow the conductive material inside the storage part 320 to be discharged to the skin of the animal through the opening. At this time, the position of the electrode part 220 is identifiable by the character pattern 130.

When the conductive material 321 is discharged and reaches the skin of the animal, the skin of the animal and the electrode part 220 are electrically connected such that the electrode part 220 collects the biosignals of the animal. The collected biosignals are transmitted to an external device via the electrode part 220, the wiring part 230, and the connecting part 250.

In the above-described method for fixing and using the biosignal sensor, the order thereof may be partially changed depending on user convenience or circumstances. In addition, although the method for measuring brainwaves from the forehead of the animal has been described using an example in the above description and drawings, it is also possible to measure biosignals other than brainwaves from other body parts, as illustrated in FIG. 10.

Hereinafter, a biosignal sensor according to the second embodiment of the present invention will be described. The same reference numerals are used for components having the same form and function as those of the first embodiment, and descriptions of the same components are omitted.

The biosignal sensor according to the second embodiment of the present invention differs from that of the first embodiment in the form of the fixing band 100, as illustrated in FIG. 11 and FIG. 12.

The fixing band 100 is composed of a head fixing part 110 and an ear fixing part 120, wherein the head fixing part 110 is the same as that of the first embodiment, and the ear fixing part 120 is composed of third and fourth ear fixing parts 123 and 124.

The third ear fixing part 123 has an ear insertion port 133 formed on one side of the connecting part 140 into which an ear on one side of the animal is inserted. The ear on one side of the animal is inserted into the ear insertion port 133 and fixed.

The fourth ear fixing part 124 has the same shape and function as the second ear fixing part 122 of the first embodiment.

The ear fixing part 120 may have a groove 120a formed concavely toward the connecting part 140 from which the third and fourth ear fixing parts 123 and 124 branch off. The groove 120a may allow the third and fourth ear fixing parts 123 and 124 to bend more flexibly to improve close-contact with the skin when they are bent while respectively wrapping around the ears on the respective sides.

Thus, compared to the first embodiment, the operation of fixing the first ear fixing part 121 to the first head fixing part 111 may be replaced with the operation of inserting an ear into the ear insertion port 133. Specifically, a user inserts the ear on one side into the ear insertion port 133, then fixes the head fixing part 110 so as to surround and wrap around the head and the chin of the animal, and then fixes the fourth ear fixing part 124 by wrapping around an ear on the other side therewith and coupling the same to the head fixing part 110.

In the above-described method for fixing and using the biosignal sensor, the order thereof may be partially changed depending on user convenience or circumstances.

The present invention described above can be implemented in various other forms without departing from the technical spirit or main features thereof. Therefore, the above embodiments are merely illustrative in all respects and should not be construed as limiting.

### INDUSTRIAL APPLICABILITY

The present invention is a biosignal sensor that collects biosignals, including brain waves, from a body part of an animal, including a human, and is available for use in the healthcare industry or medical industry.

## Claims

1. A biosignal sensor that collects biosignals, including brain waves, from a hairy body part of an animal, including a human, the biosignal sensor **characterized by** comprising:
an electrode assembly fixed to the body part, and provided with a plurality of electrodes disposed toward the skin of the animal; and
a conductive material storage body that electrically connects the plurality of electrodes to the skin of the animal by means of a conductive material.

2. The biosignal sensor according to claim 1, **characterized by** further comprising a fixing band surrounding the body part to bring the plurality of electrodes into close contact with the skin of the animal,
wherein the electrode assembly is disposed on a rear surface of the fixing band, the rear surface being in a direction toward the skin of the animal.

3. The biosignal sensor according to claim 1, **characterized in that**
the electrode assembly comprises:
a sheet part coupled to the rear surface of the fixing band;
an electrode part disposed on a rear surface of the sheet part and composed of a plurality of electrodes that collect the biosignals of the animal;
a wiring part composed of a plurality of wires, each having one end electrically connected to each of the plurality of electrodes;
an insulating part that insulates the wiring part; and
a connecting part electrically connected to the other end of each of the plurality of wires to electrically transmit the collected biological signals to the outside.

4. The biosignal sensor according to claim 3, **characterized in that**
the sheet part is detachably attached to the fixing band.

5. The biosignal sensor according to claim 1, **characterized in that** the conductive material storage body comprises:
a main body part provided with a coupling surface coupled to a rear surface of the electrode assembly, and a contact surface in contact with the skin of the animal in a direction opposite to the coupling surface; and
a plurality of storage parts each disposed at a position corresponding to the position of each of the plurality of electrodes, and provided with a plurality of internal spaces and openings formed through the main body part such that the electrode part is toward the skin of the animal,
wherein the conductive material is embedded in each of the internal spaces of the plurality of storage parts.

6. The biosignal sensor according to claim 5, **characterized in that** the storage part is made of an elastic material, such that, when elastic deformation occurs due to an external force, the conductive material is discharged from the opening of the storage part onto the skin.

7. The biosignal sensor according to claim 5, **characterized in that** the storage part is formed such that a cross-sectional diameter of the opening is smaller than a cross-sectional diameter of the internal space.

8. The biosignal sensor according to claim 7, **characterized by** further comprising a porous foam inserted into the internal space of the storage part, and having a porous structure that absorbs the conductive material to maintain a stored state.

9. The biosignal sensor according to claim 5, **characterized by** further comprising one or more partition wall portions formed around the plurality of storage parts by protruding from a bottom surface of the main body part.

10. The biosignal sensor according to claim 5, **characterized by** further comprising one or more guide parts formed at a lower portion of any one of the plurality of storage parts by protruding from the bottom surface of the main body part.

11. The biosignal sensor according to claim 5, **characterized by** further comprising a plurality of close-contact pads, each having a front surface attached to an upper surface around the opening of each of the storage parts and a rear surface attached to the skin of the animal.

12. The biosignal sensor according to claim 11, **characterized in that** the close-contact pad covers a portion of the opening of the storage part.

13. The biosignal sensor according to claim 1, **characterized in that** the electrode assembly comprises:
an electrode part disposed on the rear surface of the fixing band and composed of a plurality of electrodes that collect the biosignals of the animal;
a wiring part disposed on the rear surface of the fixing band, and each having one end electrically connected to each of the plurality of electrodes;
an insulating part that insulates the wiring part; and
a connecting part electrically connected to the other end of each of the plurality of wires to electrically transmit the collected biological signals to the outside.
